(19) Europäisches Patentamt
European Patent Office
Office européen des brevets

(11) Veröffentlichungsnummer: **0 088 299**
**B1**

(12) # EUROPÄISCHE PATENTSCHRIFT

(45) Veröffentlichungstag der Patentschrift: **12.06.85**

(51) Int. Cl.⁴: **C 07 C 97/07**, C 07 C 101/34, C 07 C 131/00, A 01 N 35/06, A 01 N 37/44

(21) Anmeldenummer: **83101838.7**

(22) Anmeldetag: **25.02.83**

(54) **Cyclohexan-1,3-dionderivate, Verfahren zu ihrer Herstellung und ihre Verwendung zur Bekämpfung unerwünschten Pflanzenwuchses.**

(30) Priorität: **04.03.82 DE 3207768**
**25.05.82 DE 3219490**

(43) Veröffentlichungstag der Anmeldung:
**14.09.83 Patentblatt 83/37**

(45) Bekanntmachung des Hinweises auf die Patenterteilung:
**12.06.85 Patentblatt 85/24**

(84) Benannte Vertragsstaaten:
**DE FR GB IT**

(56) Entgegenhaltungen:
**CHEMICAL ABSTRACTS, Band 90, Nr. 25, 18. Juni 1979, Seite 576, Nr. 203557t, Columbus, Ohio, USA**
**Perkow, Wirksubstanzen der Pflanzenschutz- und Schädlingsbekämpfungsmittel, Verlag Paul Parey, Berlin und Hamburg 1971/82**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Jahn, Dieter, Dr., Burgunderweg 8, D-6803 Neckarhausen (DE)**
Erfinder: **Becker, Rainer, Dr., Sonnenwendstrasse 83a, D-6702 Bad Duerkheim (DE)**
Erfinder: **Goetz, Norbert, Dr., Schoefferstrasse 25, D-6520 Worms (DE)**
Erfinder: **Siegel, Hardo, Dr., Hans-Purrmann-Allee, D-6720 Speyer (DE)**
Erfinder: **Wuerzer, Bruno, Dr., Dipl.-Landw., Ruedigerstrasse 13, D-6701 Otterstadt (DE)**

**EP 0 088 299 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Cyclohexan-1,3-dionderivate, Verfahren zu ihrer Herstellung sowie Herbizide, die diese Verbindungen als Wirkstoffe enthalten.

Es ist bekannt, 2-[1-Alkyl(alkenyl)oxyaminoalkyliden]cyclohexan-1,3-dionderivate zur selektiven Bekämpfung von unerwünschten monokotylen und dikotylen Pflanzen in breitblättrigen Kulturen anzuwenden. Beispielsweise ist bekannt, dass entsprechende, in 5-Stellung durch Cycloalkylreste substituierte Cyclohexan-1,3-dionderivate (JP-OS Nr. 79/19945) bzw. das Natriumsalz des 2-(1-Alkyloxyaminobutyliden)-4-methoxycarbonyl-5,5-dimethylcyclohexan-1,3-dion (Perkow, „Wirksubstanzen der Pflanzenschutz- und Schädlingsbekämpfungsmitte", Verlag Paul Parey, Berlin und Hamburg 1971/82, „Alloxydim") zu diesem Zweck verwendet werden können.

Es wurde gefunden, dass Cyclohexan-1,3-dionderivate der Formel

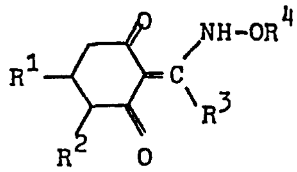

(I)

in der

$R^1$ einen durch Chlor, Brom, Alkyl oder Alkoxy mit jeweils 1 bis 3 Kohlenstoffatomen oder Phenyl einfach oder mehrfach substituierten Cycloalkylrest mit 3 bis 6 Kohlenstoffatomen oder einen gegebenenfalls durch Chlor, Brom, Alkyl oder Alkoxy mit jeweils 1 bis 4 Kohlenstoffatomen oder Phenyl einfach oder mehrfach substituierten, gegebenenfalls ein- bis vierfach olefinisch ungesättigen Cycloalkyl-, Bicycloalkyl- oder Tricycloalkylrest mit 7 bis 12 Kohlenstoffatomen,

$R^2$ Wasserstoff, Methoxycarbonyl oder Ethoxycarbonyl,

$R^3$ Alkyl mit 1 bis 4 Kohlenstoffatomen, und

$R^4$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogensubstituenten oder Propargyl bedeuten,

eine gute herbizide Wirkung gegen zahlreiche Pflanzenarten aus der Familie der Gräser (Gramineen) haben. Gleichzeitig zeigen die neuen Verbindungen ein hohes Mass an Verträglichkeit für breitblättrige und sonstige, nicht zu den Gramineen zählenden Kulturen. Darüber hinaus besitzen gewisse Verbindungen noch eine Selektivität für einzelne, auch zu den Gräsern gehörende Kulturpflanzen, wie Reis oder Weizen.

Die Verbindungen der Formel I können in mehreren tautomeren Formen auftreten, die alle vom Patentanspruch umfasst werden

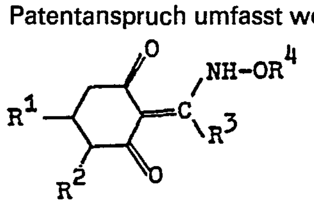

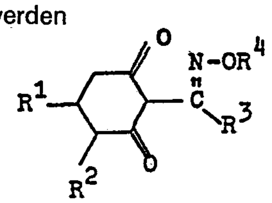

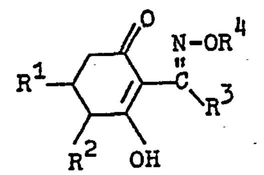

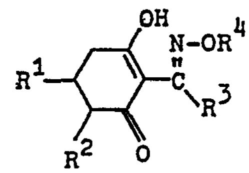

$R^1$ in Formel I bedeutet einen Cycloalkyl-, Bicycloalkyl- oder Tricycloalkylrest mit 7 bis 12 Kohlenstoffatomen, beispielsweise Cycloheptyl, Cyclooctyl, Cyclononyl, Cyclodecyl, Cycloundecyl, Cyclododecyl, Bicycloheptyl, wie Bicyclo-[2.2.1]heptyl-2, Bicyclooctyl, Tricyclooctyl, Bicyclononyl, Tricyclononyl, Bicyclodecyl, Tricyclodecyl, Bicyclododecyl, Tricyclododecyl, oder einen Cycloalkenyl-, Bicycloalkenyl- oder Tricycloalkenylrest mit 7 bis 12 Kohlenstoffatomen, wie Cycloalkenyl-, Cycloalkadienyl-, Cycloalkatrienyl- oder Cycloalkatetraenylreste sowie die entsprechenden bi- und tricyclischen Reste mit 1 bis 3 Doppelbindungen, beispielsweise Cycloheptenyl, Cyclooctenyl, Cyclooctadienyl, Cyclononenyl, Cyclodecenyl, Cyclododecenyl, Cyclododecadienyl, wie Cyclododecadien-1,5-yl-9, Cyclododecatetraenyl, wie Cyclododecatetraen-1,3,5,7-yl-10, Bicycloheptenyl, wie Bicyclo-[2.2.1]hepten-2-yl-5, Bicyclooctenyl, Bicyclononenyl, Bicyclodecenyl, Tricyclodecenyl, Bicyclododecadienyl.

Diese Reste können durch Chlor, Brom, Alkyl mit 1 bis 4 Kohlenstoffen, beispielsweise Methyl, Alkoxy mit 1 bis 4 Kohlenstoffatomen, beispielsweise Methoxy, oder Phenyl einfach oder mehrfach substituiert sein. Beispiele für solche Reste für $R^1$ sind 3,7,7-Trimethylbicyclo[4.1.0]heptyl-4, 2,6,6-Trimethylbicyclo[3.1.1]heptyl-3, 7,7-Dichlorbicyclo[4.1.0]heptyl-3, 4-Methyl-7,7-dichlorobicyclo[4.1.0]heptyl-3, Tricyclo[5.2.$0^{2,6}$]decen-8-yl-4, Tricyclo[5.2.1.$0^{2,6}$]decen-8-yl-3, 7,7-Dibrombicyclo[4.1.0]heptyl-3.

$R^1$ kann ausserdem einen durch Chlor, Brom, Alkyl mit 1 bis 3 Kohlenstoffatomen, beispielsweise Methyl, Alkoxy mit 1 bis 3 Kohlenstoffatomen, beispielsweise Methoxy, oder Phenyl einfach oder mehrfach substituierten Cycloalkylrest mit 3 bis 6 Kohlenstoffatomen bedeuten, beispielsweise 2,2-Dichlorcyclopropyl, 3-Phenyl-2,2-dichlorcyclopropyl, 4-Methoxycyclohexyl, 2,2,6-Trimethylcyclohexyl, 4-Chlorcyclohexyl, 2-Phenyl-4-methylcyclohexyl.

$R^3$ in Formel I steht für unverzweigte oder verzweigte Alkylreste mit 1 bis 4 Kohlenstoffatomen, d. h. für Methyl, Ethyl, n-Propyl, i-Propyl, n-Butyl, sek.-Butyl, i-Butyl, tert.-Butyl.

Reste für $R^4$ in Formel I sind Propargyl, Alkyl mit 1 bis 3 Kohlenstoffatomen, Alkenyl mit 3 oder 4 Kohlenstoffatomen oder Halogenalkenyl mit 3 oder 4 Kohlenstoffatomen, das bis zu drei Halogensubstituenten enthält, beispielsweise Methyl, Ethyl, n-Propyl, i-Propyl, n-Butyl, sek.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,2-Trichlorprop-1-en-3-yl.

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze in Betracht.

Die Verbindungen der Formel I können durch Umsetzung von Verbindungen der Formel

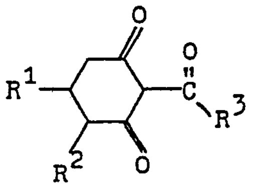

(II)

in der $R^1$, $R^2$ und $R^4$ die obengenannten Bedeutungen haben, mit Hydroxylaminderivaten $R^4O-NH_3Y$, in der $R^4$ die obengenannten Bedeutungen hat und Y ein Anion bedeutet, erhalten werden.

Man führt die Reaktion zweckmässigerweise in heterogener Phase in einem inerten Verdünnungsmittel bei einer Temperatur zwischen 0 und 80 oder zwischen 0° C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Ausserdem können auch organische Basen, wie Pyridin oder tertiäre Amine, Verwendung finden.

Die Umsetzung verläuft besonders gut in einem pH-Bereich von 2 bis 8, insbesondere von 4,5 bis 5,5. Die Einstellung des pH-Bereichs erfolgt zweckmässigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 mol, bezogen auf die Ammoniumverbindung der Formel $R^4O-NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können ausserdem durch Umsetzen der Verbindungen der Formel II mit Hydroxylaminen der Formel $R^4O-NH_2$, in der $R^4$ die obengenannten Bedeutungen hat, in inerten Verdünngsmittel bei einer Temperatur zwischen 0° C und dem Siedepunkt des Reaktionsgemisches, insbesondere zwischen 15 und 70° C, erhalten werden. Gegebenenfalls kann das Hydroxylamin als wässerige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wässerige Lösung oder in einem organischen Lösungsmittel, wie Methanol, Ethanol, Aceton, erhalten werden. Auch Natrium- und Kaliumalkoholate können als Basen dienen.

Die anderen Metallsalze, z. B. die Mangan-, Kupfer-, Zink-, Eisen-, Calcium-, Magnesium- und Bariumsalze können aus den Natriumsalzen durch Reaktion mit den entsprechenden Metallchloriden in wässerigen Lösung hergestellt werden.

Die Verbindungen der Formel II können aus Cyclohexan-1,3-dionen der Formel III, die auch in den tautomeren Formeln IIIa und IIIb vorliegen können,

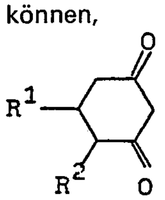

(III)

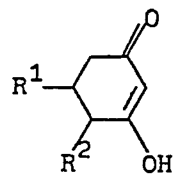

(IIIa)

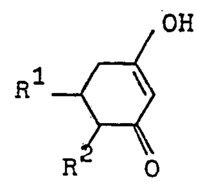

(IIIb)

nach literaturbekannten Methoden („Tetrahedron Letters", *29*, 2491 [1975]) hergestellt werden.

Es ist auch möglich, Verbindungen der Formel II über die Zwischenstufe der Enolester, die bei der Umsetzung von Verbindungen der Formel II eventuell als Isomerengemische anfallen und in Gegenwart von Imidazol- oder Pyridinderivaten umgelagert werden (JP-OS Nr. 79/063052), herzustellen.

Zu den Verbindungen der Formel III gelangt man nach literaturbekannten Verfahren, wie dies aus folgendem Schema hervorgeht

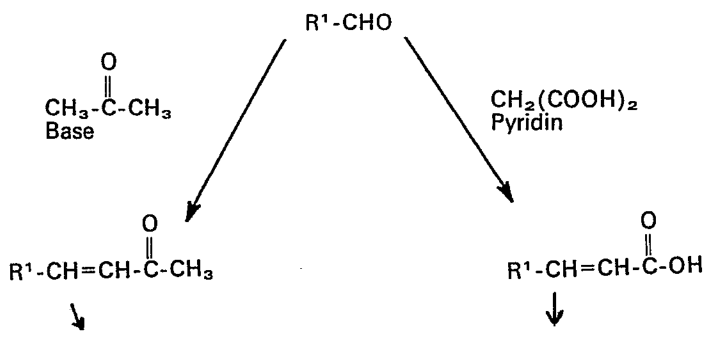

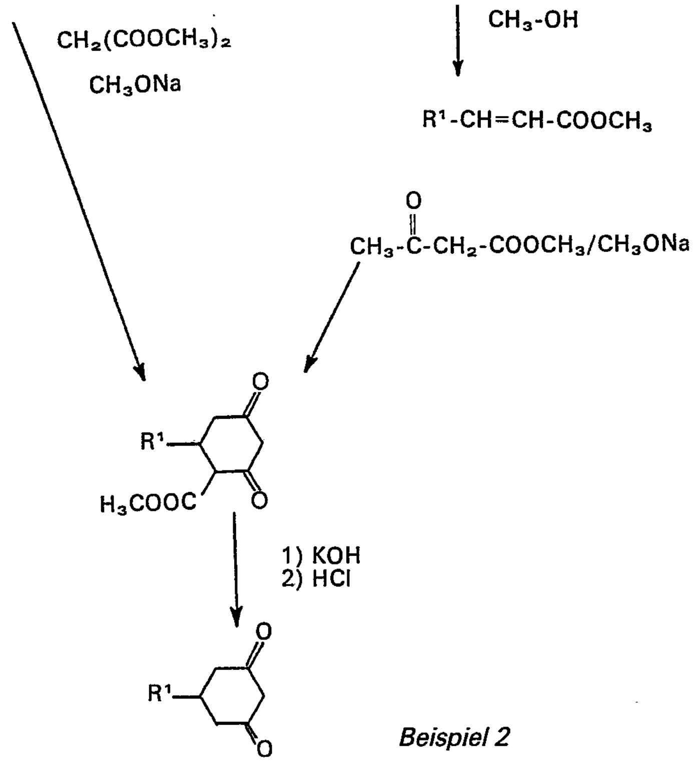


Die folgenden Beispiele erläutern die Herstellung der Cyclohexan-1,3-dionderivate der Formel I.

In den Beispielen verhalten sich Gewichtsteile zu Volumenteilen wie Kilogramm zu Liter.

*Beispiel 1*

4,9 Gew.-Teile 2-Butyryl-5-cyclooctylcyclohexan-1,3-dion, 1,1 Gew.-Teile Ethoxyamin und 80 Vol.-Teile Ethanol werden bei Raumtemperatur 12 h gerührt. Das Lösungsmittel wird unter vermindertem Druck abdestilliert, der Rückstand in 150 Teilen Dichlormethan aufgenommen, die Lösung zweimal mit Wasser gewaschen, über Natriumsulfat getrocknet und das Lösungsmittel unter vermindertem Druck abdestilliert. Man erhält 2-(1-Ethoxyaminobutyliden)-5-cyclooctylcyclohexan-1,3-dion in 94%iger Ausbeute als Öl (Verbindung Nr. 1); $n_D^{33}$: 1,5234.

*Analyse* für $C_{20}H_{33}NO_3$ (335)

| | | | |
|---|---|---|---|
| Berechnet: | C 71,6 | H 9,9 | N 4,2 |
| Gefunden: | C 72,0 | H 9,9 | N 4,1 |

*Beispiel 2*

7,5 Gew.-Teile 2-Proprionyl-5-(2,6,6-trimethylbicyclo[3.1.1]heptyl-3)-cyclohexan-1,3-dion, 2,9 Gew.-Teile Allyloxyammoniumchlorid, 2,2 Gew.-Teile wasserfreies Natriumacetat und 80 ml Ethanol werden 12 h bei Raumtemperatur gerührt. Das Lösungsmittel wird unter vermindertem Druck abdestilliert, der Rückstand wird mit 120 Teilen Wasser und 100 Teilen Methylenchlorid gerührt, die organische Phase abgetrennt, die wässerige Phase mit 50 Teilen Methylenchlorid extrahiert, die vereinigten organischen Phasen mit Wasser gewaschen, über Natriumsulfat getrocknet und unter vermindertem Druck eingeengt.

Man erhält 2-(1-Allyloxyaminopropyliden)-5-(2,6,6-trimethylbicyclo[3.1.1]hept-3-yl)cyclohexan-1,3-dion als Öl in 91%iger Ausbeute. (Verbindung Nr. 2); $n_D^{26}$: 1,5317.

*Analyse* für $C_{22}H_{33}NO_3$ (360)

| | | | |
|---|---|---|---|
| Berechnet: | C 73,5 | H 9,2 | N 3,9 |
| Gefunden: | C 73,9 | H 9,1 | N 3,8 |

Die folgenden Verbindungen erhält man in gleicher Weise

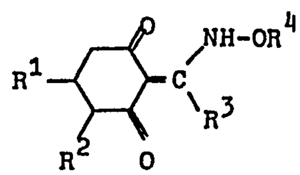

(I)

| Nr. | $R^1$ | $R^2$ | $R^3$ | $R^4$ | $n_D$ | Temperatur (°C) |
|---|---|---|---|---|---|---|
| 3 | Cycloheptyl | $COOCH_3$ | n-Propyl | Ethyl | 1,5172 | (23) |
| 4 | Cycloheptyl | $COOCH_3$ | n-Propyl | Allyl | 1,5210 | (23) |
| 5 | Cycloheptyl | H | n-Propyl | Ethyl | 1,5240 | (20) |
| 6 | Cycloheptyl | H | n-Propyl | Allyl | 1,5291 | (23) |

| Nr. | $R^1$ | $R^2$ | $R^3$ | $R^4$ | $n_D$ | Temperatur (°C) |
|---|---|---|---|---|---|---|
| 7 | Cycloheptyl | H | Ethyl | Ethyl | 1,5292 | (20) |
| 8 | Cycloheptyl | H | Ethyl | Allyl | 1,5340 | (23) |
| 9 | Cycloheptyl | H | Ethyl | 3-Chlorallyl | | |
| 10 | Cyclooctyl | H | Propyl | Allyl | 1,5281 | (33) |
| 11 | Cyclooctyl | H | Propyl | 3-Chlorallyl | | |
| 12 | Cyclooctyl | H | Ethyl | Ethyl | 1,5322 | (16) |
| 13 | Cyclooctyl | H | Ethyl | Allyl | 1,5368 | (16) |
| 14 | Cycloocten-1-yl-5 | H | n-Propyl | Ethyl | 1,5345 | (23) |
| 15 | Cycloocten-1-yl-5 | H | n-Propyl | Allyl | 1,5390 | (23) |
| 16 | Cycloocten-1-yl-5 | $COOCH_3$ | n-Propyl | Ethyl | | |
| 17 | Cycloocten-1-yl-5 | $COOCH_3$ | n-Propyl | Allyl | | |
| 18 | Cyclododecyl | H | n-Propyl | Ethyl | | |
| 19 | Cyclododecyl | H | n-Propyl | Allyl | | |
| 20 | Cyclododecadien-1,5-yl-9 | H | n-Propyl | Ethyl | 1,5290 | (31) |
| 21 | Cyclododecadien-1,5-yl-9 | H | n-Propyl | Allyl | 1,5332 | (31) |
| 22 | 7,7-Dichlorbicyclo[4.1.0]heptyl-3 | H | n-Propyl | Ethyl | 1,5444 | (18) |
| 23 | 7,7-Dichlorbicyclo[4.1.0]heptyl-3 | H | n-Propyl | Allyl | 1,5499 | (18) |
| 24 | 7,7-Dichlorbicyclo[4.1.0]heptyl-3 | H | Ethyl | Allyl | 1,5535 | (18) |
| 25 | 7,7-Dichlorbicyclo[4.1.0]heptyl-3 | H | Ethyl | Ethyl | 1,5491 | (18) |
| 26 | 3,7,7-Trimethylbicyclo[4.1.0]heptyl-4 | H | n-Propyl | Ethyl | 1,5236 | (19) |
| 27 | 3,7,7-Trimethylbicyclo[4.1.0]heptyl-4 | H | n-Propyl | Allyl | 1,5286 | (19) |
| 28 | Bicyclo[2.2.1]heptyl-2 | H | n-Propyl | Allyl | | |
| 29 | Bicyclo[2.2.1]heptyl-2 | H | n-Propyl | Ethyl | | |
| 30 | Bicyclo[2.2.1]hepten-2-yl-5 | H | n-Propyl | Ethyl | 1,5334 | (22) |
| 31 | Bicyclo[2.2.1]hepten-2-yl-5 | H | n-Propyl | Allyl | 1,5399 | (22) |
| 32 | 2,6,6-Trimethylbicyclo[3.1.1.]heptyl-3 | H | n-Propyl | Allyl | 1,5286 | (22) |
| 33 | 2,6,6-Trimethylbicyclo[3.1.1]heptyl-3 | H | n-Propyl | Ethyl | 1,5242 | (26) |
| 34 | 2,6,6-Trimethylbicyclo[3.1.1]heptyl-3 | H | n-Propyl | Methyl | 1,5298 | (26) |
| 35 | 2,6,6-Trimethylbicyclo[3.1.1]heptyl-3 | $COOCH_3$ | n-Propyl | Ethyl | 1,5193 | (20) |
| 36 | 2,6,6-Trimethylbicyclo[3.1.1]heptyl-3 | $COOCH_3$ | n-Propyl | Allyl | 1,5245 | (20) |
| 37 | 2,6,6-Trimethylbicyclo[3.1.1]heptyl-3 | H | Ethyl | Ethyl | 1,5268 | (26) |
| 38 | Tricyclo[5.2.1.$0^{2,6}$]decen-8-yl-3 | H | n-Propyl | Ethyl | | |
| 39 | Tricyclo[5.2.1.$0^{2,6}$]decen-8-yl-3 | H | n-Propyl | Allyl | | |
| 40 | Tricyclo[5.2.1.$0^{2,6}$]decen-8-yl-4 | H | n-Propyl | Allyl | 1,5478 | (22) |
| 41 | Tricyclo[5.2.1.$0^{2,6}$]decen-8-yl-4 | H | n-Propyl | Ethyl | 1,5413 | (22) |
| 42 | 2,6,6-Trimethylbicyclo[3.1.1]heptyl-3 (Natriumsalz) | H | n-Propyl | Ethyl | | |
| 43 | 2,6,6-Trimethylbicyclo[4.1.1]heptyl-3 (Natriumsalz) | H | n-Propyl | Allyl | | |
| 44 | 2,2-Dichlorcyclopropyl | H | n-Propyl | Ethyl | 1,5260 | (24) |
| 45 | 2,2-Dichlorcyclopropyl | H | n-Propyl | Allyl | 1,5341 | (23) |
| 46 | 3-Phenyl-2,2-dichlorcyclopropyl | H | Ethyl | Ethyl | 1,5623 | (24) |
| 47 | 3-Phenyl-2,2-dichlorcyclopropyl | H | n-Propyl | Ethyl | 1,5522 | (27) |
| 48 | 2,2,6-Trimethylcyclohexyl | H | n-Propyl | Ethyl | 1,5079 | (31) |
| 49 | 2,2,6-Trimethylcyclohexyl | H | n-Propyl | Allyl | 1,5219 | (31) |
| 50 | 4-Methoxycyclohexyl | H | n-Propyl | Ethyl | 1,5138 | (22) |
| 51 | 4-Methoxycyclohexyl | H | n-Propyl | Allyl | 1,5215 | (22) |
| 52 | 2-Phenyl-4-methylcyclohexyl | H | n-Propyl | Allyl | | |
| 53 | 2-Phenyl-4-methylcyclohexyl | H | n-Propyl | Ethyl | 1,5512 | (25) |
| 54 | 4-Methylcyclohexyl | H | n-Propyl | Ethyl | | |
| 55 | 4-Methylcyclohexyl | H | n-Propyl | Allyl | | |
| 56 | 4-Chlorcyclohexyl | H | n-Propyl | Allyl | | |
| 57 | 4-Chlorcyclohexyl | H | n-Propyl | Ethyl | | |
| 58 | 1-Phenylcyclohexen-3-yl-6 | H | n-Propyl | Ethyl | 1,5628 | (22) |
| 59 | 1-Phenylcyclohexen-3-yl-6 | H | n-Propyl | Allyl | 1,5653 | (22) |
| 60 | Cycloheptyl | H | n-Propyl | Methyl | 1,5296 | (26) |
| 61 | Cycloheptyl | H | n-Propyl | n-Propyl | 1,5204 | (26) |
| 62 | Cycloheptyl | H | n-Propyl | Propargyl | 1,5335 | (26) |
| 63 | Bicyclo[2.2.1]hepten-2-yl-5 | H | n-Propyl | 3-Chlorallyl | 1,5480 | (26) |
| 64 | Bicyclo[2.2.1]hepten-2-yl-5 | H | n-Propyl | Propargyl | 1,5434 | (26) |

| Nr. | $R^1$ | $R^2$ | $R^3$ | $R^4$ | $n_D$ | Temperatur (°C) |
|---|---|---|---|---|---|---|
| 65 | 2,2-Dichlorcyclopropyl | $COOCH_3$ | n-Propyl | Ethyl | 3,80<br>(s; $COOCH_3$),<br>4,15<br>(q; $OCH_2$) | |
| 66 | 3,7,7-Trimethylbicyclo[4.1.0]heptyl-4 | $COOCH_3$ | n-Propyl | Ethyl | 3,79<br>(s; $COOCH_3$),<br>4,12<br>(q; $OCH_2$) | |
| 67 | 6,6-Dimethylbicyclo[3.1.1]heptan-2-yl-2 | H | n-Propyl | Ethyl | 4,10<br>(q; $OCH_2$),<br>5,30<br>(s; =CH) | |
| 68 | 6,6-Dimethylbicyclo[3.1.1]heptan-2-yl-2 | H | n-Propyl | Allyl | 2,90<br>N-<br>‖<br>(t;-C-$CH_2$),<br>4,55<br>(d; $OCH_2$) | |
| 69 | 6,6-Dimethylbicyclo[3.1.1]heptan-2-yl-2 | H | n-Propyl | 3-Chlorallyl | 1,535 | (24) |
| 70 | 4-tert.-Butoxycyclohexyl | H | n-Propyl | Ethyl | | |
| 71 | Cyclododecadien-1,5-yl-9 | H | n-Propyl | 3-Chlorallyl | | |
| 72 | Cyclododecadien-1,5-yl-9 | H | n-Propyl | Propargyl | | |
| 73 | Cyclododecadien-1,5-yl-9 | H | n-Propyl | n-Propyl | | |
| 74 | Cyclododecadien-1,5-yl-9 | H | Ethyl | n-Propyl | | |
| 75 | Cyclododecadien-1,5-yl-9 | H | Ethyl | Allyl | | |
| 76 | Cyclododecadien-1,5-yl-9 | H | Ethyl | 3-Chlorallyl | | |
| 77 | Cyclododecadien-1,5-yl-9 | H | Ethyl | Ethyl | | |
| 78 | Cyclododecadien-1,5-yl-9 | H | Ethyl | Propargyl | | |
| 79 | Cyclododecadien-1,5-yl-9 | H | Methyl | Propargyl | | |
| 80 | Cyclododecadien-1,5-yl-9 | H | Methyl | n-Propyl | | |
| 81 | Cyclododecadien-1,5-yl-9 | H | Methyl | Ethyl | | |
| 82 | Cyclododecadien-1,5-yl-9 | H | Methyl | Allyl | | |
| 83 | Cyclododecyl | H | n-Propyl | 3-Chlorallyl | | |
| 84 | Cyclododecyl | H | n-Propyl | Propargyl | | |
| 85 | Cyclododecyl | H | Ethyl | Ethyl | | |
| 86 | Cyclododecyl | H | Ethyl | Allyl | | |
| 87 | Cyclododecyl | H | Ethyl | 3-Chlorallyl | | |
| 88 | Cyclododecyl | H | Ethyl | Propargyl | | |
| 89 | Cyclododecatetraen-1,3,5,7-yl-10 | H | n-Propyl | Ethyl | | |
| 90 | Cyclododecatetraen-1,3,5,7-yl-10 | H | n-Propyl | Allyl | | |
| 91 | Cyclooctatrien-1,3,5-yl-7 | H | n-Propyl | Allyl | | |
| 92 | Cyclooctatrien-1,3,5-yl-7 | H | n-Propyl | Ethyl | | |
| 93 | Cyclooctadien-1,3-yl-6 | H | n-Propyl | Ethyl | | |
| 94 | Cyclooctadien-1,3-yl-6 | H | n-Propyl | Allyl | | |
| 95 | Cyclododecadien-1,5-yl-9 (Natriumsalz) | H | n-Propyl | Ethyl | | |
| 96 | Cyclododecadien-1,5-yl-9 (Natriumsalz) | H | n-Propyl | Allyl | | |
| 97 | Cycloheptyl | H | n-Propyl | Methyl | | |
| 98 | Cycloheptyl | H | n-Propyl | n-Propyl | | |
| 99 | Cycloheptyl | H | n-Propyl | Propargyl | | |
| 100 | 1,4(3)-Dimethylcyclohexen-3-yl | H | n-Propyl | Ethyl | | |

Die Cyclohexan-1,3-dionderivate der Formel I können beispielsweise in Form von direkt versprühbaren Lösungen, Pulvern, Suspensionen, auch hochprozentigen wässerigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis

hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wässerige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95, vorzugsweise zwischen 0,5 und 90 Gew.-%, Wirkstoff.

Beispiele für Formulierungen sind

I. Man vermischt 90 Gew.-Teile des Wirkstoffs Nr. 1 mit 10 Gew.-Teilen N-Methyl-α-pyrrolidon und erhält eine Lösung, die zur Anwendung in Form kleinster Tropfen geeignet ist.

II. 10 Gew.-Teile des Wirkstoffs Nr. 2 werden in einer Mischung gelöst, die aus 90 Gew.-Teilen Xylol, 6 Gew.-Teilen des Anlagerungsproduktes von 8 bis 10 mol Ethylenoxid an 1 mol Ölsäure-N-monoethanolamid, 2 Gew.-Teilen Calciumsalz der Dodecylbenzolsulfonsäure und 2 Gew.-Teilen des Anlagerungsproduktes von 40 mol Ethylenoxid an 1 mol Ricinusöl besteht.

III. 20 Gew.-Teile des Wirkstoffs Nr. 10 werden in einer Mischung gelöst, die aus 60 Gew.-Teilen Cyclohexanon, 30 Gew.-Teilen Isobutanol, 5 Gew.-Teilen des Anlagerungsproduktes von 7 mol Ethylenoxid an 1 mol Isooctylphenol und 5 Gew.-Teilen des Anlagerungsproduktes von 40 mol Ethylenoxid an 1 mol Ricinusöl besteht.

IV. 20 Gew.-Teile des Wirkstoffs Nr. 2 werden in einer Mischung gelöst, die aus 25 Gew.-Teilen Cyclohexanol, 65 Gew.-Teilen einer Mineralölfraktion vom Siedepunkt 210 bis 280° C und 10 Gew.-Teilen des Anlagerungsproduktes von 40 mol Ethylenoxid an 1 mol Ricinusöl besteht. Durch Eingiessen und feines Verteilen der Lösung in 100 000 Gew.-Teilen Wasser erhält man eine wässerige Dispersion, die 0,02 Gew.-% des Wirkstoffs enthält.

V. 80 Gew.-Teile des Wirkstoffs Nr. 2 werden mit 3 Gew.-Teilen des Natriumsalzes der Diisobutylnaphthalinsulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen.

VI. 5 Gew.-Teile des Wirkstoffs Nr. 1 werden mit 95 Gew.-Teilen feinteiligem Kaolin vermischt. Man erhält auf diese Weise ein Stäubemittel, das 5 Gew.-% des Wirkstoffs enthält.

VII. 30 Gew.-Teile des Wirkstoffs Nr. 11 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 5 werden mit 2 Teilen Calciumsalz der Dodecylbenzolsulfonsäure, 8 Teilen Fettalkoholpolyglykolether, 2 Teilen Natriumsalz eines Phenol/Harnstoff/Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Vorzugsweise werden die Wirkstoffe bzw. diese enthaltende Mittel nach dem Auflaufen der unerwünschten Pflanzen ausgebracht. Sind die Wirkstoffe für gewisse Kulturpflanzen weniger verträglich, so können auch Ausbringungstechniken angewandt werden,

bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen *(post-directed, lay-by)*.

Die Aufwandmengen an Wirkstoff betragen je nach Jahreszeit, Zielpflanzen und Wachstumsstadium 0,025 bis 5 kg/ha.

Die Wirkung der Cyclohexan-1,3-dionderivate der Formel I auf das Wachstum von Pflanzen aus der Gräserfamilie (Gramineen) und breitblättrigen Kulturpflanzen lässt sich durch Gewächshausversuche zeigen:

Als Kulturgefässe dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefässe leicht beregnet, um Keimung und Wachstum in Gang zu bringen. Danach deckt man die Gefässe mit durchsichtigen Plastikhauben ab, bis die Pflanzen angewachsen sind. Diese Abdeckung bewirkt ein gleichmässiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die für die Nachauflaufanwendung benutzten Reis- und Sojapflanzen sowie die Buschbohnen werden in einem mit Torfmull *(peat)* angereicherten Substrat angezogen. Eine Beeinträchtigung der Ergebnisse ist nicht zu befürchten, da es sich um Blattbehandlungen handelt. Zur Nachauflaufbehandlung werden entweder direkt gesäte und in den gleichen Gefässen aufgewachsene Pflanzen ausgewählt, oder sie werden erst als Keimpflanzen getrennt angezogen und einige Tage vor der Behandlung in die Versuchsgefässe verpflanzt. Die Aufwandmengen für die Nachauflaufbehandlung variieren je nach Wirkstoff. Sie betragen 0,125, 0,25, 0,5 bzw. 1,0 kg Wirkstoff/ha. Eine Abdeckung unterbleibt bei der Nachauflaufbehandlung.

Die Versuchsgefässe werden im Gewächshaus aufgestellt, wobei für wärmeliebende Arten wärmere Bereiche (20 bis 35° C) und für solche gemässigter Klimate 10 bis 25° C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

*Avena fatua* (Flughafer), *Avena sativa* (Hafer), *Beta vulgaris* (Zuckerrübe), *Bromus tectorum* (Dachtrespe), *Digitaria sanguinalis* (Blut-Fingerhirse), *Echinochloa crus-galli* (Hühnerhirse), *Glycine max.* (Sojabohnen), *Gossypium hirsutum* (Baumwolle), *Lolium multiflorum* (Ital. Raygras), *Oryza sativa* (Reis), *Phaseolus vulg.* (Buschbohnen), *Setaria italica* (Kolbenhirse), *Sorghum halepense* (Aleppohirse), *Triticum aestivum* (Weizen), *Zea mays* (Mais).

Bei der Prüfung auf herbizide Wirkung bei Vorauflaufanwendung zeigen beispielsweise die Verbindungen Nrn. 1, 2, 5, 6, 10, 13, 20, 22, 23, 24, 25, 26, 27, 32, 34, 35 und 37 bei Aufwandmengen von 3,0 kg Wirkstoff/ha eine beachtliche herbizide Aktivität gegen grasartige Pflanzen.

Bei der Prüfung auf selektive herbizide Eigenschaften bei Nachauflaufanwendung bekämpfen beispielsweise die Verbindungen Nrn. 20, 32 und 33 mit 0,125 kg bzw. 1,0 kg Wirkstoff/ha Grasarten sehr gut. Ebenso zeigt die Verbindung Nrn. 22 mit 0,25 kg Wirkstoff/ha eine gute herbizide Wirkung gegen unerwünschte grasartige Kulturpflanzen, während sie für Weizen bzw. Reis verträglich ist. Die Verbindungen Nrn. 32 und 33 sind bei Aufwandmengen von 1,0 kg Wirkstoff/ha gut verträglich für breitblättrige Kulturen, während gleichzeitig Grasarten stark geschädigt werden.

In Anbetracht der Verträglichkeit und der Vielseitigkeit der Applikationsmethoden können die erfindungsgemässen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen:

| Botanischer Name | Deutscher Name |
|---|---|
| *Allium cepa* | Küchenzwiebel |
| *Ananas Comosus* | Ananas |
| *Arachis hypogaea* | Erdnuss |
| *Asparagus officinalis* | Spargel |
| *Beta vulgaris* spp. *altissima* | Zuckerrübe |
| *Beta vulgaris* spp. *rapa* | Futterrübe |
| *Beta vulgaris* spp. *esculenta* | Rote Rübe |
| *Brassica napus* var. *napus* | Raps |
| *Brassica napus* var. *napobrassica* | Kohlrübe |
| *Brassica napus* var. *rapa* | Weisse Rübe |

| Botanischer Name | Deutscher Name |
|---|---|
| *Brassica rapa* var. *silvestris* | Rübsen |
| *Camellia sinensis* | Teestrauch |
| *Carthamus tinctorius* | Saflor: Färberdistel |
| *Carya illinoinensis* | Pekannussbaum |
| *Citrus limon* | Zitrone |
| *Citrus maxima* | Pampelmuse |
| *Citrus reticulata* | Mandarine |
| *Citrus sinensis* | Apfelsine |
| *Coffea arabica (Coffea canephora, Coffea liberica)* | Kaffee |
| *Cucumis melo* | Melone |
| *Cucumis sativus* | Gurke |
| *Daucus carota* | Möhre |
| *Elaeis guineensis* | Ölpalme |
| *Fragaria vesca* | Erdbeere |
| *Glycine max* | Sojabohne |
| *Gossypium hirsutum (Gossypium arboreum Gossypium herbaceum Gossypium vitifolium)* | Baumwolle |
| *Helianthus annuus* | Sonnenblume |
| *Helianthus tuberosus* | Topinambur |
| *Hevea brasiliensis* | Parakautschukbaum |
| *Hordeum vulgare* | Gerste |
| *Humulus lupulus* | Hopfen |
| *Ipomoea batatas* | Süsskartoffeln |
| *Juglans regia* | Walnussbaum |
| *Lactua sativa* | Kopfsalat |
| *Lens culinaris* | Linse |
| *Linum usitatissimum* | Faserlein |
| *Lycopersicon lycopersicum* | Tomate |
| *Malus* spp. | Apfel |
| *Manihot esculenta* | Maniok |
| *Medicago sativa* | Luzerne |
| *Metha piperita* | Pfefferminze |
| *Musa* spp. | Obst- und Mehlbanane |
| *Nicotiana tabacum (N. rustica)* | Tabak |
| *Olea europaea* | Ölbaum |
| *Oryza sativa* | Reis |
| *Phaseolus lunatus* | Mondbohne |
| *Phaseolus mungo* | Erdbohne |
| *Phaseolus vulgaris* | Buschbohnen |
| *Petroselinum crispum* spp. *tuberosum* | Wurzelpetersilie |
| *Picea abies* | Rotfichte |
| *Abies alba* | Weisstanne |
| *Pinus* spp. | Kiefer |
| *Pisum sativum* | Gartenerbse |
| *Prunus avium* | Süsskirsche |
| *Prunus domestica* | Pflaume |
| *Prunus duscis* | Mandelbaum |
| *Prunus persica* | Pfirsich |
| *Pyrus communis* | Birne |
| *Ribes sylvestre* | Rote Johannisbeere |
| *Ribes uva-crispa* | Stachelbeere |
| *Ricinus communis* | Rizinus |
| *Saccharum officinarum* | Zucherrohr |
| *Secale cereale* | Roggen |
| *Sasamum indicum* | Sesam |
| *Solanum tuberosum* | Kartoffel |
| *Sorghum dochna* | Zuckerhirse |
| *Spinacia oleracea* | Spinat |
| *Theobroma cacao* | Kakaobaum |
| *Trifolium pratense* | Rotklee |

| Botanischer Name | Deutscher Name |
|---|---|
| *Triticum aestivum* | Weizen |
| *Vaccinium corymbosum* | Kulturheidelbeere |
| *Vaccinium vitis-idaea* | Preisselbeere |
| *Vicia faba* | Pferdebohnen |
| *Vigna sinensis (V. unguiculata)* | Kuhbohne |
| *Vitis vinifera* | Weinrebe |

Zur Verbreiterung des Wirkungsspektrums und zur Erzielung synergistischer Effekte können die Cyclohexan-1,3-dionderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Betracht.

Ausserdem kann es von Nutzen sein, die neuen Wirkstoffe bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Cyclohexan-1,3-dionderivate der Formel

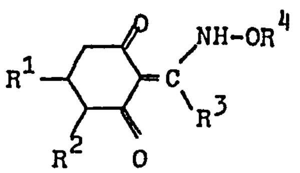

(I)

in der

$R^1$ einen durch Chlor, Brom, Alkyl oder Alkoxy mit jeweils 1 bis 3 Kohlenstoffatomen oder Phenyl einfach oder mehrfach substituierten Cycloalkylrest mit 3 bis 6 Kohlenstoffatomen oder einen gegebenenfalls durch Chlor, Brom, Alkyl oder Alkoxy mit jeweils 1 bis 4 Kohlenstoffatomen oder Phenyl einfach oder mehrfach substituierten, gegebenenfalls ein- bis vierfach olefinisch ungesättigten Cycloalkyl-, Bicycloalkyl- oder Tricycloalkylrest mit 7 bis 12 Kohlenstoffatomen,

$R^2$ Wasserstoff, Methoxycarbonyl oder Ethoxycarbonyl,

$R^3$ Alkyl mit 1 bis 4 Kohlenstoffatomen, und

$R^4$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogensubstituenten oder Propargyl bedeuten,

und Salze dieser Verbindungen.

2. 2-(1-Ethoxyaminobutyliden)-5-(cyclododeca-1,5-dien-9-yl)cyclohexan-1,3-dion.

3. 2-(1-Allyloxyaminobutyliden)-5-(cyclododeca-1,5-dien-9-yl)cyclohexan-1,3-dion.

4. Verfahren zur Herstellung eines Cyclohexan-1,3-dionderivates der Formel I gemäss Anspruch 1, dadurch gekennzeichnet, dass man eine Verbindung der Formel

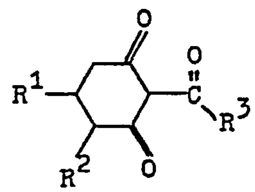

(II)

in der $R^1$, $R^2$ und $R^3$ die im Anspruch 1 gennanten Bedeutungen haben,

a) mit einer Ammoniumverbindung der Formel $R^4O-NH_3Y$, in der $R^4$ die im Anspruch 1 genannten Bedeutungen hat und Y ein Anion bedeutet, in einem inerten Verdünnungsmittel gegebenenfalls in Gegenwart von Wasser bei einer Temperatur zwischen 0 und 80° C in Gegenwart einer Base, oder

b) mit einem gegebenenfalls in wässeriger Lösung vorliegenden Hydroxylamin der Formel $R^4O-NH_2$, in der $R^4$ die im Anspruch 1 genannten Bedeutung hat, in einem inerten Lösungsmittel umsetzt.

5. Verfahren nach Anspruch 4, dadurch gekennzeichnet, dass man eine Verbindung der Formel II mit einer Ammoniumverbindung der Formel $R^4O-NH_3Y$ bei einem pH-Wert im Bereich zwischen 2 und 8 umsetzt.

6. Herbizid, enthalten ein Cyclohexan-1,3-dionderivat der Formel I gemäss Anspruch 1.

7. Herbizid, enthaltend 2-(1-Ethoxyaminobutyliden)-5-(cyclododeca-1,5-dien-9-yl)cyclohexan-1,3-dion.

8. Herbizid, enthaltend 2-(1-Allyloxyaminobutyliden)-5-(cyclododeca-1,5-dien-9-yl)cyclohexan-1,3-dion.

9. Herbizid, enthaltend inerte Zusatzstoffe und ein Cyclohexan-1,3-dionderivat der Formel I gemäss Anspruch 1.

10. Verfahren zur Bekämpfung unerwünschten Pflanzenwachstums, dadurch gekennzeichnet, dass man die unerwünschten Pflanzen und/oder von unerwünschtem Pflanzenwachstum freizuhaltende Flächen mit einer herbizid wirksamen Menge eines Cyclohexan-1,3-dionderivates der Formel I gemäss Anspruch 1 behandelt.

## Claims

1. A cyclohexane-1,3-dione derivative of the formula

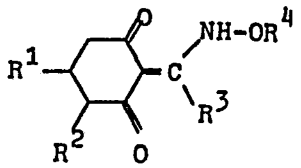

(I)

where

$R^1$ is cycloalkyl of 3 to 6 carbon atoms which is monosubstituted or polysubstituted by chlorine, bromine, alkyl or alkoxy, each of 1 to 3 carbons atoms, or by phenyl, or is a cycloalkyl, bicycloalkyl or tricycloalkyl radical of 7 to 12 carbon atoms which is unsubstituted or monosubstituted or polysubstituted by chlorine, bromine, alkyl or alkoxy, each of 1 to 4 carbon atoms, or by phenyl, and is optionally mono- to tetraolefinically unsaturated,

$R^2$ is hydrogen, methoxycarbonyl or ethoxycarbonyl,

$R^3$ is alkyl of 1 to 4 carbon atoms, and

$R^4$ is alkyl of 1 to 3 carbon atoms, alkenyl of 3 or 4 carbon atoms, haloalkenyl of 3 or 4 carbon atoms and 1 to 3 halogen atoms, or propargyl,

and salts thereof.

2. 2-(1-Ethoxyaminobutylidene)-5-(cyclododeca-1,5-dien-9-yl)-cyclohexane-1,3-dione.

3. 2-(1-Allyloxyaminobutylidene)-5-(cyclododeca-1,5-dien-9-yl)-cyclohexane-1,3-dione.

4. A process for the preparation of a cyclohexane-1,3-dione derivative of the Formula I as claimed in Claim 1, wherein a compound of the formula

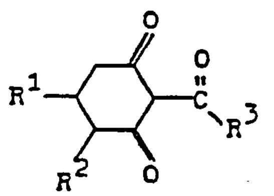

(II)

where $R^1$, $R^2$ and $R^3$ have the meanings given in Claim 1, is reacted

(*a*) with an ammonium compound of the formula $R^4O-NH_3Y$, where $R^4$ has the meanings given in Claim 1 and Y is an anion, in an inert diluent in the presence or absence of water and in the presence of a base at a temperature of from 0 to 80° C, or

(*b*) with a hydroxylamine optionally in the form of an aqueous solution and having the formula $R^4O-NH_2$, where $R^4$ has the meanings given in Claim 1, in an inert solvent.

5. A process as claimed in Claim 4, wherein a compound of the Formula II is reacted with an ammonium compound of the formula $R^4O-NH_3Y$ at a pH of from 2 to 8.

6. A herbicide containing a cyclohexane-1,3-dione derivative as claimed in Claim 1.

7. A herbicide containing 2-(1-ethoxyaminobutylidene)-5-(cyclododeca-1,5-dien-9-yl)-cyclohexane-1,3-dione.

8. A herbicide containing 2-(1-allyloxyaminobutylidene)-5-(cyclododeca-1,5-dien-9-yl)-cyclohexane-1,3-dione.

9. A herbicide containing inert additives and a cyclohexane-1,3-dione derivative of the Formula I as claimed in Claim 1.

10. A process for combating the growth of unwanted plants, wherein the unwanted plants and/or the areas to be kept free from unwanted plant growth are treated with a herbicidally effective amount of a cyclohexane-1,3-dione derivative of the Formula I as claimed in Claim 1.

## Revendications

1. Dérivés de cyclohexan-1,3-dione de formule

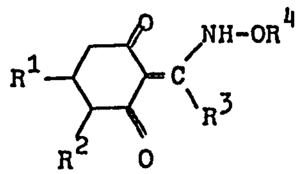

(I)

dans laquelle

$R^1$ représente un reste cycloalkyle ayant 3 à 6 atomes de carbone, substitué une ou plusieurs fois par chlore, brome, alkyle ou alcoxy ayant chacun 1 à 3 atomes de carbone ou par phényle, ou bien un reste cycloalkyle, bicycloalkyle ou tricycloalkyle de 7 à 12 atomes de carbone, éventuellement insaturé oléfiniquement de une à quatre fois, substitué éventuellement par chlore, brome, alkyle ou alcoxy ayant chacun 1 à 4 atomes de carbone, ou par phényle,

$R^2$ représente hydrogène, méthoxycarbonyle ou éthoxycarbonyle,

$R^3$ alkyle ayant 1 à 4 atomes de carbone, et

$R^4$ alkyle ayant 1 à 3 atomes de carbone, alcényle ayant 3 ou 4 atomes de carbone, halogénalkyle ayant 3 ou 4 atomes de carbone et 1 à 3 substituants halogène ou propargyle,

et des sels de ces composés.

2. 2-(1-éthoxyaminobutylidène)-5-(cyclododéca-1,5-dién-9-yl)-cyclohexan-1,3-dione.

3. 2-(1-allyloxyaminobutylidène)-5-(cyclododéca-1,5-dién-9-yl)-cyclohexan-1,3-dione.

4. Procédé de préparation d'un dérivé de cyclohexan-1,3-dione de formule I selon la revendication 1, caractérisé par le fait que l'on fait réagir un composé de formule

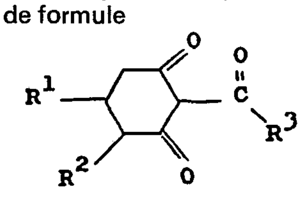

(II)

dans laquelle $R^1$, $R^2$ et $R^3$ ont les significations indiquées dans la revendication 1,

a) avec un dérivé d'ammonium de formule $R^4O-NH_3Y$, dans laquelle $R^4$ a les significations indiquées dans la revendication 1 et Y représente un anion, dans un diluant inerte, éventuellement en présence d'eau, à une température comprise entre 0 et 80° C, en présence d'une base, ou

b) avec une hydroxylamine, se trouvant éventuellement en solution aqueuse, de formule $R^4O-NH_2$, dans laquelle $R^4$ a les significations indiquées dans la revendication 1, dans un solvant inerte.

5. Procédé selon la revendication 4, caractérisé par le fait que l'on fait réagir un composé de formule II avec un composé d'ammonium de formule $R^4O-NH_3Y$, avec une valeur de pH dans la zone comprise entre 2 et 8.

6. Herbicide, contenant un dérivé de cyclohexan-1,3-dione de formule I selon la revendication 1.

7. Herbicide contenant de la 2-(1-éthoxyaminobutylidène)-5-(cyclododéca-1,5-dién-9-yl)-cyclohexan-1,3-dione.

8. Herbicide contenant de la 2-(1-allyloxyaminobutylidène)-5-(cyclododéca-1,5-dién-9-yl)-cyclohexan-1,3-dione.

9. Herbicide contenant des additifs inertes et un dérivé de cyclohexan-1,3-dione de formule I selon la revendication 1.

10. Procédé pour lutter contre une croissance de plantes indésirables, caractérisé par le fait que l'on traite les plantes indésirables et/ou des surfaces à maintenir exemptes de croissance de plantes indésirables, avec une quantité efficace, au point de vue herbicide, d'un dérivé de cyclohexan-1,3-dione de formule I selon la revendication 1.